(19) Europäisches Patentamt
European Patent Office
Office européen des brevets

(11) **EP 2 665 411 B1**

(12) **EUROPEAN PATENT SPECIFICATION**

(45) Date of publication and mention
of the grant of the patent:
**23.05.2018 Bulletin 2018/21**

(51) Int Cl.:
*A61B 5/026* (2006.01)  *A61B 5/024* (2006.01)
*A61B 5/0285* (2006.01)  *A61B 5/00* (2006.01)

(21) Application number: **12736086.5**

(22) Date of filing: **13.01.2012**

(86) International application number:
**PCT/FI2012/050031**

(87) International publication number:
**WO 2012/098289 (26.07.2012 Gazette 2012/30)**

(54) **A METHOD AND SYSTEM FOR VISUALIZATION OF CARDIOVASCULAR PULSATION WAVES**

VERFAHREN UND SYSTEM ZUR VISUALISIERUNG KARDIOVASKULÄRER IMPULSWELLEN

PROCÉDÉ ET SYSTÈME DE VISUALISATION D'ONDES DE PULSATION CARDIOVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2011 FI 20115053**

(43) Date of publication of application:
**27.11.2013 Bulletin 2013/48**

(73) Proprietor: **Delfin Technologies Oy**
**70211 Kuopio (FI)**

(72) Inventors:
• **KAMSHILIN, Alexei**
**FI-00530 Helsinki (FI)**
• **MIRIDONOV, Serguei**
**22860 (MX)**

(74) Representative: **Berggren Oy, Tampere**
**Visiokatu 1**
**33720 Tampere (FI)**

(56) References cited:
**WO-A2-2007/122375    US-A- 5 747 789**
**US-A- 5 983 120**

• **HU S. ET AL.: 'Feasibility of Imaging Photoplethysmography' INTERNATIONAL CONFERENCE ON BIOMEDICAL ENGINEERING AND INFORMATICS vol. 2, 27 May 2008, pages 72 - 75, XP031275837**
• **HU S. ET AL.: 'A study of opto-physiological modeling to quantify tissue absorbance in imaging photoplethysmography' 32ND ANNUAL INTERNATIONAL CONFERENCE OF THE IEEE ENGINEERING IN MEDICINE AND BIOLOGY SOCIETY 31 August 2010, pages 5776 - 5779, XP031795175**

**Description**

The field of the invention

The invention relates to a method for visualization of cardiovascular pulsation waves according to preamble of claim 1. The invention relates also to a system according to preamble of claim 11 for application of the method according to the invention.

Background of the invention

Visualization of blood-flow dynamics in different parts of a human body is extremely important in medicine because it might be capable to provide supportive information needed for diagnosis of numerous diseases. Visualization of blood flow assists the examination of general and altered microcirculatory characteristics. It is believed, for example, that improved clinical observation of the microcirculation of human organs would be extremely useful in assessing states of shock such as septic, hypovolemic, cardiogenic and obstructive shock in patients and in guiding resuscitation therapies aimed at correcting this condition. In particular, it has been found that the active recruitment of the microcirculation maybe an important component of resuscitation. Additionally, improved clinical observation of the status of microcirculation would yield useful complementary information in circulatory abnormalities related to local or systemic pathologies such as tumors, dermatological diseases and conditions, traumas such as burns, surgery and surgical complications, drugs, therapeutic effects, many general diseases such as diabetes and cardiovascular diseases.

Numerous optical systems and methods which use the light reflected from the human skin for analysis of changes in blood volume, blood pulsatility, and tissue perfusion by blood have been proposed. All of them are based on analysis of the light spectrum scattered and absorbed inside the human skin. The reflectance from full thickness skin is dependent upon the optical properties of skin structure including the blood-free epidermis, as well as the dermis. The thickness of epidermis including the stratum corneum is 10-150 $\mu$m. The dermis lying beneath the epidermis has a complicated structure including elastin and collagen fibers and blood vessels of different sizes, sweat glands, sebaceous glands, and hair follicles. The thickness of the dermal layer is 1-4 mm. The epidermal layer contributes about 6% to the total reflectance mainly due to specular reflection, which allows neglecting the influence of the epidermis on formation the skin images in the reflected light. Since *in vivo* dermis layer contains blood, its optical properties are modulated during important physiological cycles such as cardiac and respiratory. Therefore, if light is projected onto an area of skin and the emergent light is detected after its interaction with the skin, blood and other tissue, time varying changes of light intensity having a relation with blood volume, known as the plethysmogram, can be observed. The difference of light absorption spectra between oxygenated hemoglobin and deoxygenated hemoglobin is the physical basis for the optical oximeters which provide information about the arterial blood oxygen saturation. The optical oximeters are used nowadays in many settings: hospital, outpatient, domiciliary use, and in veterinary clinics [1,2]. A photoplethysmograph device characterized by the improved signal-to-noise ratio is disclosed in document [3]. However, this device measures the blood volume as a function of time either in a single point or in several points of human body and does not visualize dynamics of the blood perfusion in space.

Formation of the space-resolved images of the photoplethysmograph signal under illumination of the skin by light at several wavelengths and recording the series of images in reflected light into a video camera was recently reported in papers [4,5]. However, these systems visualize the average distribution of the oxygenation saturation over the observation area of a human body but do not provide information about dynamics of this process.

There are various optical systems and methods which record and process the images obtained in the reflection light for visualization of different parameters of blood flow in the dermis. Publication [6] describes the optical system for estimation of hemoglobin concentration in a blood vessel when the subject is illuminated by light sources from different positions. Again this system provides average measuring of the hemoglobin, not its dynamics during the biological cycle.

The method and apparatus for reflected imaging analysis is disclosed in the US-patent [7]. The system can be used to determine such characteristics as the hemoglobin concentration per unit volume of blood, the number of white blood cells per unit volume of blood, a mean cell volume, the number of platelets per unit volume of blood, and the hematocrit. The optical system is to be configured so as to capture the image reflected from the illuminated object at a depth less than a multiple scattering length. This requirement imposes serious limitations on selection of the wavelength of illuminating light and does not allow use of the near-infrared light, which is characterized by large penetration length in the human skin. Moreover, the method fails in visualization of both temporal and spatial dynamics of these parameters.

Several improvements of the reflected imaging systems were described aimed to increase reliability of the biological information obtained from recorded images at different wavelengths. One non-invasive method for *in-vivo* analysis of a vascular system is disclosed in the PCT application [8]. In this method the reflected spectral images of a microcirculatory system are processed to measure the volume and concentration of blood vessels, including arteries, veins and capillaries. In this method, the series of the images is taken and used for selecting and screening only high-quality images which are then used for estimation of mean image intensity and motion blur parameters. Similar method for analysis of spectral images of a microcirculatory system to measure blood characteristics with slightly different approach of spectral images screening is disclosed in the patent application [9].

Another method for *in-vivo* analysis is disclosed in the patent application [10]. The method is based on the fact that the spectrum of blood-related chromophores (which are moving in the blood vessels) is temporally different from the spectrum of the non-moving objects. In this method, the spectra of the moving objects are obtained by acquiring a time series of images at several wavelengths, and by eliminating the contribution of the stationary spectra. These separated spectra are then decomposed into the absorption spectra of oxy- and deoxyhemoglobin for estimation of the blood oxygenation. However, the method works correctly only if the reflected images are captured from a depth that is less than the multiple scattering length.

Document US5747789 discloses the preamble of claim 1.

Common disadvantage of all of the above techniques for *in-vivo* analysis of a vascular system is that they assess the averaged parameters of blood perfusion and do not visualize dynamic physiological changes. Thus, there is a need in the art for a method and device that provides visualization of dynamic changes of the blood perfusion *in-vivo* and non-invasively during physiological cycles (such as cardiac and respiratory cycles). There is a further need for device and method that allows visualization of the relative phase and amplitude of blood pulsations in spatially different parts of a human body.

Summary of the invention

The inventive idea of the invention is to process series of digital images (frames) captured from desired area of the skin of the human body such that a series of output images represents the dynamics of blood-pulsation waves during one cycle of the periodical physiological process in a living body. The processing of the captured series of images includes steps of multiplying each frame of the series of images with a reference function synchronized with a periodical physiological process of the living body, forming a correlation image by summarizing the respective pixels over all the images of the series after the multiplication with the reference function and forming the output images from the correlation images. To put it more precisely, the method according to the invention is characterized in what will be presented in the characterizing part of the claim 1 and the system according to the invention is characterized in what will be presented in the characterizing part of the claim 11.

The method and the system according to the invention provide significant advantages to the prior art. With the method and system according to present invention it is possible to visualize the dynamic changes of blood-volume variations spatially during such physiological phenomena as e.g. cardiac pulsation or respiratory cycle. This improves possibilities for application of the examination of blood microcirculatory characteristics among others in cases in which the present known methods and devices, such as disclosed in the document [3], have not been adequate For instance, by means of the visualization method and system according to the invention a medical doctor is able to get supportive cardiovascular information to assist diagnosis making in such situations as e.g. in local or systemic pathologies such as tumors, shock, dermatological diseases and conditions, traumas such as burns, surgery and surgical complications, drugs, therapeutic effects, many general diseases such as diabetes and cardiovascular diseases. Researchers in biology, physiology, medicine, and cosmetics will benefit of using the method and system according to the invention since it provides possibility to study quantitatively dynamic reactions of living body on various impacts such as drugs, ointments, posture, exposure to ionizing or non-ionizing radiation, ageing, ambient conditions etc. through analysis of visualized cardiovascular pulsation waves.

Brief description of the drawings

Preferred embodiments of the present invention are now described with reference to the attached drawings wherein,

Figure 1 shows a generic schematic of the system for visualization of the phase and amplitude of blood perfusion in a human body,
Figure 2 shows an example of an image captured by a camera chip under illumination of a palm by the light at the wavelength of 780 nm and cross-polarization filtering,
Figure 3 shows a block-diagram wherein it is schematically described how the processing of recorded series of images is performed in the method according to the invention,
Figure 4 shows a typical evolution of the pixel intensity function $U_1(t)$ in time after removing of its mean value,
Figure 5 shows typical spectrum of the signal after application of the fast Fourier transform to the function $U_1(t)$.
Figure 6 shows schematically the multiplication of the series of images by the reference function $R_c(t)$, and
Figure 7 shows a block-diagram of a second embodiment wherein it is schematically described the steps needed for formation of a video which represents dynamics of blood-volume variations during the cardiac cycle and steps needed for visualization of blood-volume changes during the respiratory cycle when the reference signal Rc(t) or Re(t) is generated by means of an additional sensor.

Detailed description of the invention

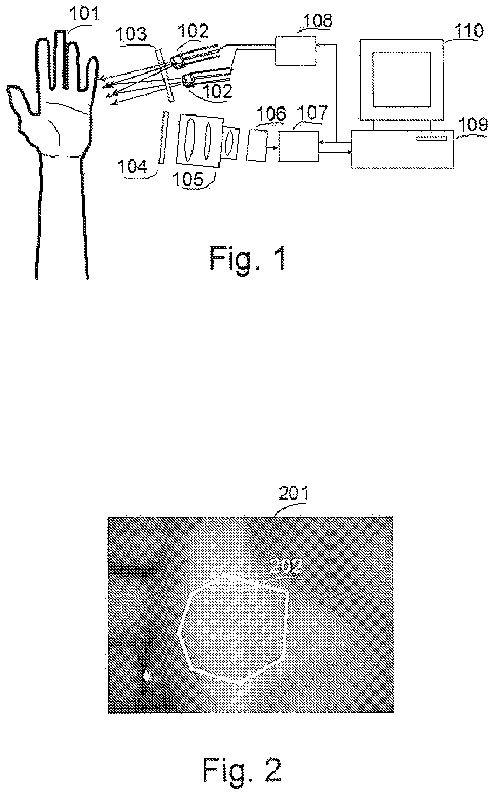

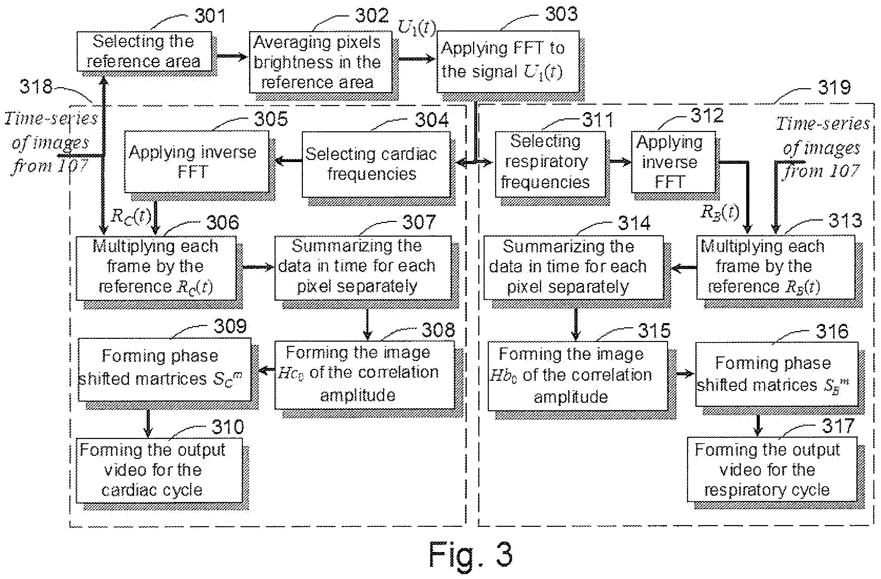

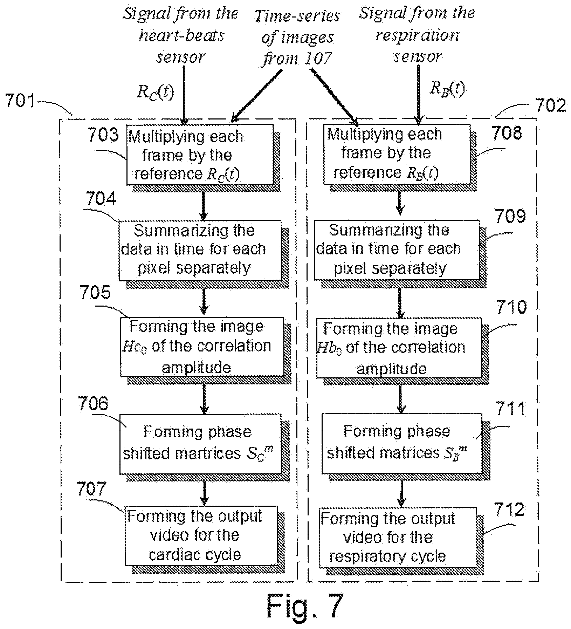

Figure 1 shows a generic schematic of the system for visualization of the phase and amplitude of the blood perfusion in a human body. The system shown in figure 1 comprises a light source 102 that is configured to illuminate a part of a living body 101 by which cardiovascular pulsation waves are to be visualized, an image-capturing unit 106 with an optical means 103-105 for collecting the light reflected from that part of the living body 101 and for forming a focused image of the living body in to the image-capturing unit 106 (with memory chip 107), a processing unit 109 for further processing of the captured image, a display unit 110 for displaying at least the output images i.e. the images formed in the processing unit 109 as a result of the further processing. The system comprises also a communication link being connected between the image-capturing unit, the processing unit 109 and the display unit 110. Furthermore, the system according to figure 1 comprises a computer program being assembled in to the processing unit 109 in order to carry out data processing steps (such as shown in figures 3 and 7) being necessary to form output images showing the dynamic changes of the amplitude of the fractional blood volume and spatial distribution of the relative phase of these oscillations in the living body during a cardiovascular cycle.

A part 101 of the human body is illuminated by a light source 102 which generates the light at the certain wavelength. The light source 102 can be in this embodiment either single light-emitting diode (LED) or an array of LEDs. Laser-diodes or lasers can be also used as a light source instead of LEDs. Different LEDs or lasers in the light source array can operate either at the same wavelengths or at different wavelengths. The illumination can be either continuous or pulsed. When the illumination is pulsed it is preferably synchronized with the moment of electronic read-out of the information in the camera chip 106.

Before illuminating the part of the living body 101, the light can be polarized after passing through a polarization filter 103. The light reflected from the part 101 of the human body is collected into an image-capturing unit 106 being in this embodiment a light-sensitive camera chip (it can be either CCD camera chip or CMOS camera chip which provides transformation of the received optical flux into an electrical signal representing two-dimensional electronic image) by means of an optical lens or objective 105. The objective 105 provides focused imaging of the part 101 of the human body at the sensitive area of the camera chip 106. When using with polarized illumination, the reflected light passes through another polarization filter 104 before it is captured by the camera chip 106. It is known from [3] that the DC offset caused by tissue surface reflections will reduce the dynamic range of the photoplethysmograph. Therefore it is of benefit to filter out the light reflected from the surface, which can be done by means of polarizing filters 103 and 104. A polarizing filter transmits only light polarized along a given axis. This polarization state is retained when light is reflected but lost when light is scattered. If the light incident on the tissue is polarized, the light reflected at the surface retains this polarization state and can be attenuated by a second polarizing filter (in figure 1 reference 104) orientated at 90 degrees to the linear polarization state of the incident light. However, light that penetrates the tissue and is scattered by the blood and other media loses its polarization and hence passes through the orthogonally oriented polarizing filter 104, and is detected by the camera chip 106. Nevertheless, the system can also operate without polarizing filters 103 and 104.

The camera chip 106 provides recording a series of frames of the part of the human body 101 with the frame rate which exceeds the heart beat rate at least 2 times (preferably at least 3 times) into the memory chip 107. Recorded series of frames is transferred via the communication link for further processing into the processing unit 109 that is in this case a personal computer. The communication link between the different parts of the apparatus may be, for instance, a communication link known as such (e.g. an USB cable, Bluetooth link or WLAN network etc.) that is able to transfer the image data appropriately between the different parts of the system.

An example of a frame 201 captured by the camera chip 106 under illumination of a palm by the light at the wavelength of 780 nm and cross-polarization filtering is shown in figure 2. During capturing of the frame series, the person was asked to avoid any movement of his body and to keep normal breathing. Therefore, all frames in the recorded series are very similar one to another so that differences between them are hardly being visible by a naked eye. However, after processing these frames in a computer, time-variable parts of the frames are revealed and enhanced.

The recorded series of frames is processed in the way schematically described in the block-diagram shown in figure 3. In the step 301 it is selected an area of the frame which will serve for formation of the reference signal. Hereinafter this area is referred to as reference area. Alternatively, the reference signal may be generated by using an electrical signal from additional sensors of heart beats and/or respiration as it will be described later. The shape, size, and position of the reference area can be chosen arbitrarily but preferably parts of image not related to the human body should be excluded from the reference area. An example of the reference area 202 is shown in figure 2 as an area limited by the polygon.

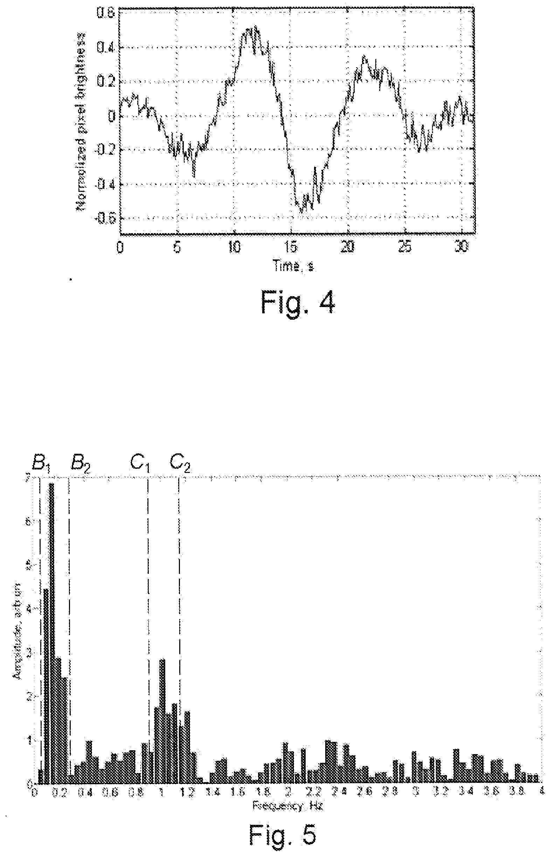

Then in the step 302, the values assigned to the pixels (pixels intensity) included in the reference area 202 are summarized and normalized to the averaged magnitude of these pixels. Thus for each frame from the recorded series of frames is obtained a single number, which is variable from one frame to another forming a function $U_1(t)$ where t is the moment of frame capturing. Typical evolution of the function $U_1(t)$ in time after removing of its mean value is shown in figure 4 .

According to the established theoretical model confirmed by the experimental data [11], the physiological processes of heart pulsations and breathings lead to modulation in time of the blood fractional volume at the respective frequencies. Modulation of the blood volume results in the absorption modulation of the light penetrated in the skin, which leads to the intensity modulation of the light reflected *in vivo* from a human body. Therefore, the function $U_1(t)$ is modulated both at the heart beat rate and at the respiration rate as it shown in figure 4. In the case when the intensity modulation is not clearly revealed in the function $U_1(t)$, the steps 301 and 302 can be repeated for another reference area of the recorded images.

In the step 303, the fast Fourier transfer is applied to the function $U_1(t)$ for estimation of the heart beat rate and the respiratory rate. Typical spectrum of the signal is shown in figure 5. Instabilities of the rates of heart beats and respiration during the time of the recording result in broadening of the frequencies representative for the cardiac pulsation and breathing.

In the step 304, a band of frequencies representative for the heart beats (which are usually in the range between 0.7 and 2 Hz) is selected. An example of the selected band is shown by vertical bars $C_1$ and $C_2$ in the figure 5.

After the frequency band corresponding to the heart beats is selected, all other frequencies are truncated, and the inverse fast Fourier transfer is applied exclusively to the truncated spectrum (for frequencies f in the range of $C_1 > f > C_2$) in the next step 305. This mathematical operation reconstructs the reference function $R_C(t)$ which represents the heart pulsations. Note that $R_C(t)$ has both the real and imaginary parts. After calculation of inverse Fourier transform it is normalized in such a way that

$$\sum_t \text{Re}[R_C(t)]R_C(t) = 1. \qquad (1)$$

Here $\text{Re}[R_C(t_1)]$ denotes real part of $R_C(t_1)$. This reference function is further used for lock-in amplification (synchronous detection) of the recorded series of frames. The normalization allows further to evaluate the mean complex amplitude of the signal synchronized with the heart beat within the chosen frequency range at every pixel of the frame.

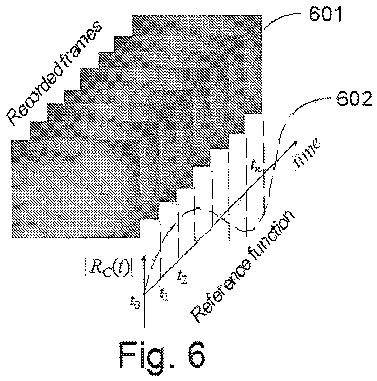

In the step 306, the series of frames is multiplied by the reference function in such a way that the intensity of each pixel of the first frame is multiplied by the same coefficient $R_C(t = t_0)$ where $t_0$ is the moment when the first frame was captured. Then the second frame is multiplied by the coefficient $R_C(t_1)$ where $t_1$ is the moment of the second frame capturing, and so on. Schematically the step 306 of multiplication of the series of frames 601 by the reference function $R_C(t)$ (602) is shown in figure 6. Note that in this figure it is plotted only the real part of the function $R_C(t)$ to simplify the drawing but the function $R_C(t)$ has both the real and imaginary parts. Therefore, after multiplication, it is obtained the series of frames in which the intensity of pixels has the complex numbers.

Correlation matrix $S_C(x,y)$ is calculated in the next step 307 by summarizing over all frames the intensity of the pixels having the same coordinates (x,y) multiplied with the reference function calculated in the previous step 306 in accordance with:

$$S_C(x, y) = \sum_t I(x, y, t) R_C(t), \qquad (2)$$

where I(x,y,t) is a frame from the series of frames 601 captured at the moment of t. The correlation matrix $S_C(x,y)$ contains the same number of pixels as any of the initial frames I(x,y,t) (which contain only positive values of pixels intensity) but the pixels values in the matrix become complex because the reference function $R_C(t)$ is complex.

As seen from Eq.2, the matrix $S_C(x,y)$ is approximately equal to the cross-correlation function of the reference $R_C(t)$ and the time-varying image of a part of human body. Since $R_C(t)$ represents only the cardiac pulsations, the matrix $S_C(x,y)$ is a lock-in amplification of the pixels whose intensity varies in time synchronously with the heart beats. Different pixels in the matrix $S_C(x,y)$ may have different amplitude of intensity oscillations and different relative phase. This difference becomes visible in the step 308, where the image $Hc_0(x,y)$ is formed by calculating the real part of the distribution $S_c$ as

$$Hc_0(x, y) = \text{Re}[S_C(x, y)]. \qquad (3)$$

Image $Hc_0(x,y)$ shows instant deviation of the fractional blood volume oscillations at the heart-beats frequency occurred in the pixel with the coordinates of (x,y) from its mean value at the time when the phase of the reference function $\Phi_{Rc}=0$. Dynamic changes of the amplitude and phase of the fractional blood volume oscillations during the cardiac cycle are visualized by calculating of the correlation matrix of the recorded series of frames 601 with the phase-shifted reference function $R_C(t)$:

$$S_C^m(x, y) = \sum_t I(x, y, t) R_C(t) \exp(i\phi_m) = S_C(x, y) \exp(i\phi_m) \quad (4)$$

Here the phase $\Phi_m$ takes the values at least from 0 to $2\pi$. The real part of the phase-shifted correlation matrix is an image which shows the spatial distribution of the relative difference of the fractional blood volume, which occurs at the moment of $t_{Cm} = \phi_m/(2\pi f_C)$, where $f_C$ is the mean rate of the heart beats. By gradually changing the phase $\Phi_m$, dynamic changes of the amplitude of the oscillating part of the fractional blood volume in the skin are reconstructed simultaneously in all observable points (x,y) during the cardiac cycle. A series of images $Hc_m(x,y)$, which shows dynamic variations of the fractional blood volume, is calculated in the step 309 as

$$Hc_m(x, y) = \mathrm{Re}[S_C(x, y)]\cos(\phi_m) + \mathrm{Im}[S_C(x, y)]\sin(\phi_m). \quad (5)$$

The values assigned to the pixels of images $Hc_m(x,y)$ can be positive, negative, or zero. Zero level means either absence of the blood-volume oscillations at the heart beats or oscillations phase shifted by 90° in respect to the reference function $R_C(t)$. Positive values are oscillations of the blood volume in phase with $R_C(t)$, while negative values are in counter phase. The higher the amplitude of the oscillations, the larger value is assigned to the pixel. The output video is formed in the step 310 from the series of images $Hc_m(x,y)$ calculated using Eq.5 for different phase $\Phi_m$ which are sequentially running in the range from 0 to $2\pi$ For the convenience, positive amplitude of the blood-volume oscillations can be marked by one color in the video frames while the negative is marked by another color. The video shows dynamic changes of the amplitude of the fractional blood volume in the skin during the cardiac cycle and spatial distribution of the relative phase of these oscillations.

Processing of the recorded series of frames for visualization of blood perfusion dynamics during the respiration cycle is carried out in a similar way like for the cardiac cycle. The steps of this processing are grouped within the modulus 319 in figure 3. As seen, all the steps are generally the same as in the modulus 318 which describes the lock-in amplification of time-variable parts of the images synchronized with the heart beats. The only difference is that in the step 311 a frequency band representative to the respiration rate (which is usually in the range between 0.05 and 0.3 Hz) is selected from the spectrum of the function $U_1(t)$. The band representative for the breathing is marked by the vertical bars $B_1$ and $B_2$ in the spectrum of $U_1(t)$ shown in figure 5. Thereafter, the reference function $R_B(t)$ is reconstructed in the step 312 by applying inverse fast Fourier transfer to the truncated spectrum containing only the frequencies in the range of $B_1 > f > B_2$ In the step 313, each frame I(x,y,t) is multiplied by the reconstructed reference function $R_B(t)$ similarly as it was done in the step 306. Then in the step 314, the correlation matrix $S_B(x,y)$ is calculated as

$$S_B(x, y) = \sum_t I(x, y, t) R_B(t) \quad (6)$$

The matrix $S_B(x,y)$ is a lock-in amplification of the pixels whose intensity varies in time synchronously with the breathing. Image $Hb_0(x,y)$ is calculated in the step 315 as a real part of the correlation matrix $S_B(x,y)$:

$$Hb_0(x, y) = \mathrm{Re}[S_B(x, y)] \quad (7)$$

Image $Hb_0(x,y)$ shows instant deviation of the fractional blood volume oscillations at the breathing frequency occurred in the pixel with the coordinates of (x,y) from its mean value at the time when the phase of the reference function $\Phi_{RB}=0$. Series of the phase-shifted correlation matrices $S_B^m(x, y)$ are formed in the step 316 as a product of the matrix $S_B(x,y)$ and the phase of $\exp(i\phi_m)$ with $\Phi_m$ taking values at least from 0 to $2\pi$:

$$S_B^m(x, y) = S_B(x, y)\exp(i\phi_m) \qquad (8)$$

The real part of the phase-shifted correlation matrix is an image which shows the spatial distribution of the relative difference of the fractional blood volume occurring at the moment of $t_{Bm} = \varphi_m/(2\pi f_B)$ Here $f_B$ is the mean rate of the breathing. By gradually changing the phase $\Phi_m$, dynamic changes of the amplitude of the oscillating part of the fractional blood volume in the skin are reconstructed simultaneously in all observable points (x,y) during the breathing cycle. Images $Hb_m$ (x, y) are calculated in the step 317 as

$$Hb_m(x, y) = \mathrm{Re}[S_B(x, y)]\cos(\phi_m) + \mathrm{Im}[S_B(x, y)]\sin(\phi_m) \qquad (9)$$

The output video is formed in the step 317 from the series of images $Hb_m(x,y)$ calculated using eq.9 for different phase $\Phi_m$ which are sequentially running in the range from 0 to $2\pi$ The video shows dynamic changes of the amplitude of the fractional blood volume in the skin during the respiratory cycle and spatial distribution of the relative phase of these oscillations. Frame processing in the modulus 318 and 319 can be performed either in a parallel or sequential mode.
In a second embodiment of the invention the reference function [either $R_c(t)$ or $R_B(t)$] is generated from an additional sensor of either heart beats or respiration. During the recording of a series of images of the part 101 of the human body by the camera chip 106, the output signals from the heart beats and/or respiratory sensor are recorded in the personal computer 109. Recording of the series of frames should be synchronized with the recording of the reference signals from the external sensors so that the amplitude of the electrical signal of the sensor is saved in the computer only once per each recorded frame. Thereafter, the recorded series of images is processed as it is schematically described in the block-diagram shown in figure 7. The modulus 701 of figure 7 shows the steps needed for formation of a video which represents dynamics of blood-volume variations during the cardiac cycle, while the modulus 702 shows the steps needed for visualization of blood-volume changes during the respiratory cycle.
Data processing within these modules 701 and 702 is executed similarly as within the modules 318 and 319, respectively. The processing starts from the multiplication of each recorded frame I(x,y,t) by the reference function of either cardiac cycle $R_C(t)$ or respiratory cycle $R_B(t)$ (steps 703 and 708, respectively). Thereafter in steps 704 and 709, the correlation matrices $S_c(x,y)$ and $S_B(x,y)$ are calculated by summarizing over all frames the intensity of the pixels having the same coordinates (x,y) multiplied with the respective reference function [either $R_C(t)$ or $R_B(t)$]. Images $Hc_m(x,y)$ and $Hb_m(x,y)$, which show spatial distribution of the fractional blood volume oscillating at the frequency of heart beats and breathing, are calculated in the steps 705 and 710, respectively.
Two series of the phase-shifted correlation matrices are formed in steps 706 and 711 as multiplication of the respective matrices $S_C(x,y)$ and $S_B(x,y)$ by the phase of with $\Phi_m$ running at least from 0 to $2\pi$. The series of images are calculated in steps 707 and 712 as the real part of the respective matrices and thus forming two videos which show dynamic changes of the amplitude of the fractional blood volume in the skin during the cardiac and respiratory cycle, respectively.
In a third embodiment of the invention dynamic changes of hemoglobin concentration during either cardiac or respiratory cycle are visualized. In this case the illumination of the object 101 is executed in the pulsed regime by at least 2 different LEDs emitting light at different central wavelengths. The wavelengths of the LEDs are chosen to have high absorption by deoxygenated hemoglobin (Hb) at the red wavelength, and oxygenated hemoglobin (HbO$_2$) at the infrared wavelength, thus the ratio of absorption at the two wavelengths is proportional to the concentrations of Hb and HbO$_2$, and hence oxygen saturation. Typically, the wavelength of one LED is chosen to be shorter than 800 nm while another is longer than 800 nm. Note that the wavelength of 800 nm is so called isobestic wavelength at which absorption caused by Hb and HbO$_2$ in human body is the same. Illumination of the object 101 is synchronized with electronic readout of the information from the camera chip 106. By sequential switching between emission bands of the red and infrared LEDs, images of the object at these different wavelengths are acquired in an alternating fashion. Synchronized switching of the LEDs is executed by means of the LED controller 108.
During the processing of the acquired series of images, the images at one wavelength are separated from those at another wavelength. Each series of images is then processed in the similar way as it is described in the first preferred embodiment. After the spatial distribution of the amplitude of blood volume oscillations is calculated for the series of images at each wavelength, their ratio is proportional to arterial oxygen saturation with the proportionality coefficients which are derived from the experimental comparison with a conventional pulse oximeter sensor.
The invention is not limited to the above-presented example embodiments, but it may vary within the scope of the inventive idea presented in the appended claims.

References

1. P. A. Kyriacou, "Pulse oximetry in the oesophagus," Physiol. Meas. 27, R1-R35 (2006).

2. J. Allen, "Photoplethysmography and its application in clinical physiological measurement," Physiol. Meas. 28, R1-R40 (2007).

3. J. Crowe, M. Grubb, B. Hayes-Gill, and N. Miles, "Photoplethysmography," Patent No. WO 2007/122375 (2007).

4. K. Humphreys, T. Ward, and C. Markham, "Noncontact simultaneous dual wavelength photoplethysmography: a further step toward noncontact pulse oximetry," Rev. Sci. Instrum. 78, 044304-1-044304-6 (2007).

5. F. P. Wieringa, F. Mastik, and A. F. W. van der Steen, "Contactless multiple wavelength photoplethysmographic imaging: a first step toward "SpO2 camera" technology," Ann. Biomed. Eng. 33, 1034-1041 (2005).

6. US2008/081968; S. Numada and T. Ozawa, "Nonivasive living body measuring device and noninvasive living body measuring method".

7. US 5,983,120; W. Groner and R. G. Nadeau, "Method and apparatus for reflected imaging analysis".

8. WO0215786; C. A. Cook, M. K. Emresoy, and H. Farid, "System, method and computer program product for measuring blood properties form a spectral image".

9. WO 02/15788; E. P. Krotkov, Y. Wang, Z. Zhong, and R. M. Danen, "System, method and computer program product for screening a spectral image".

10. US 2005/131284; A. Grinvald, D. Nelson, and I. Vanzetta, "Characterization of moving objects in a stationary background".

11. W. Cui, L. E. Ostrander, and B. Y. Lee, "In vivo reflectance of blood and tissue as a function of light wavelength," IEEE Trans. Biomed. Eng. 37, 632-639 (1990).

**Claims**

1.  A method for visualization of cardiovascular pulsation waves comprising following method steps of :

    - illuminating with a light source (102) the living body (101) with light penetrating through the skin of the body (101) for interacting via absorption and/or scattering with a vascular system of the living body (101),
    - collecting the light reflected from the living body in the form of a focused frame (201, 601) into an image capturing unit (106, 107),
    - capturing a series of frames (201, 601) by the image capturing unit (106, 107);

    **characterized in that** it further comprises the steps of:

    - multiplying by means of a processing unit (109) the frames (201, 601) of the series of frames by a reference function (602) synchronized with a periodical physiological process of the body (101),
    - forming by means of the processing unit (109) a correlation image by summarizing respective pixels multiplied by the reference function (602) over the frames (201, 601) of the series of frames,
    - calculating by means of the processing unit (109) an output image representing dynamics of blood pulsation waves in the living body (101) from the correlation images as function of the phase of the periodical physiological process of the body (101).

2.  The method according to claim 1, **characterized in that** the periodical physiological process of the body (101) are the heart beats.

3. The method according to claim 1, **characterized in that** the periodical physiological process of the body (101) is the breathing.

4. The method according to any of the preceding claims 1 to 3, **characterized in that** the reflecting light of the light source (102) is filtered by means of at least two polarizing filters (103, 104) which, the first polarizing filter (103) is positioned between the light source (102) and the living body (101), and the second polarizing filter (104) is positioned between the illuminated living body (101) and the image-capturing unit (106, 107).

5. The method according to any of the preceding claims 1 to 4, **characterized in that**, frames (201, 601) are captured by the image capturing device (106, 107) having frame rate at least two times higher than the highest rate of a quasi-periodical physiological process of the living body.

6. The method according to any of the preceding claims 1 to 5, **characterized in that** the reference function (602) is formed after analysis of average intensity oscillations of the series of frames (201, 601).

7. The method according to any of the preceding claims 1 to 5, **characterized in that**, the reference function (602) is formed from an external signal representing the periodical physiological process.

8. The method according to either claim 6 or claim 7, **characterized in that** the output image is formed from series of frames (201, 601) and that every frame of the output image is formed by forming spatial distribution of a phase-shifted correlation image which is the real part of the correlation image multiplied with a different exponential-phase coefficient.

9. The method according to claim 8, **characterized in that** in the output image it is shown spatial distribution of the amplitude of the correlation image.

10. The method according to claim 9, **characterized in that** the spatial distribution of the amplitude in the output image is coded by means of pseudo-colors representing the relative phase of the phase-shifted correlation image.

11. A system for visualization of blood-pulsation waves in a living body comprising at least one light source (102) configured to illuminate the living body (101), an image-capturing unit (106, 107) with an optical means (105) for collecting the light reflected from the living body (101) and for forming a focused image (201, 601) of the living body (101) in to the image-capturing unit (106, 107), a processing unit (109) a display unit (110), and a communication link between the image-capturing unit (106, 107), the processing unit (109) and the display unit (110), **characterized in that** the processing unit (109) comprises means for receiving the series of frames (201, 601) captured by the image-capturing unit (106, 107) and that the processing unit (109) comprises means for processing the series of frames (201, 601) according to any of the claims 1 to 8.

12. The system according to claim 11, **characterized in that** the light source (102) is a light source generating light in visible, near infrared, or infrared region of the spectrum.

13. The system according to claim 11, **characterized in that** the light source (102) is a light source generating light in the pulse regime at different wavelengths synchronously with the frame grabber of the image capturing device (106, 107).

**Patentansprüche**

1. Ein Verfahren zur Visualisierung kardiovaskulärer Pulsationswellen, welches die folgenden Verfahrensschritte umfasst:

- Beleuchten des lebenden Körpers (101) mit einer Lichtquelle (102) mit Licht, welches die Haut des Körpers (101) zur Interaktion durch Absorption und/oder Streuung mit einem Gefäßsystem des lebenden Körpers (101) durchdringt,
- Sammeln des von dem lebenden Körper reflektierten Lichts in Form eines fokussierten Einzelbildes (201, 601) in einer Bilderfassungseinheit (106, 107),
- Erfassen einer Reihe von Einzelbildern (201, 601) durch die Bilderfassungseinheit (106, 107),

**dadurch gekennzeichnet, dass** es darüber hinaus die folgenden Schritte umfasst:

- Vervielfachen, mittels einer Verarbeitungseinheit (109), der Einzelbilder (201, 601) der Reihe von Einzelbildern durch eine Referenzfunktion (602), die mit einem periodischen physiologischen Vorgang des Körpers (101) synchronisiert ist,
- Bilden eines Korrelationsbildes mittels der Verarbeitungseinheit (109) durch Zusammenfassen entsprechender Pixel, die durch die Referenzfunktion (602) über die Einzelbilder (201, 601) der Reihe von Einzelbildern multipliziert wurden,
- Berechnen, mittels der Verarbeitungseinheit (109), einer Ausgangs-Bilddarstellungsdynamik von Pulsationswellen des Blutes in dem lebenden Körper (101) aus den Korrelationsbildern als Funktion der Phase des periodischen physiologischen Vorgangs des Körpers (101).

2. Verfahren gemäß Anspruch 1, **dadurch gekennzeichnet, dass** der periodische physiologische Vorgang des Körpers (101) die Herzschläge sind.

3. Verfahren gemäß Anspruch 1, **dadurch gekennzeichnet, dass** der periodische physiologische Vorgang des Körpers (101) die Atmung ist.

4. Verfahren gemäß einem der vorhergehenden Ansprüche 1 bis 3, **dadurch gekennzeichnet, dass** das reflektierende Licht der Lichtquelle (102) mittels mindestens zwei Polarisationsfilter (103, 104) gefiltert wird, wobei sich der erste Polarisationsfilter (103) zwischen der Lichtquelle (102) und dem lebenden Körper (101) befindet und sich der zweite Polarisationsfilter (104) zwischen dem beleuchteten lebenden Körper (101) und der Bilderfassungseinheit (106, 107) befindet.

5. Verfahren gemäß einem der vorhergehenden Ansprüche 1 bis 4, **dadurch gekennzeichnet, dass** Einzelbilder (201, 601) durch die Bilderfassungsvorrichtung (106, 107) mit einer Einzelbildrate erfasst werden, die mindestens zweimal höher als die höchste Rate eines quasi-periodischen physiologischen Vorgangs des lebenden Körpers ist.

6. Verfahren gemäß einem der vorhergehenden Ansprüche 1 bis 5, **dadurch gekennzeichnet, dass** die Referenzfunktion (602) nach Analyse der durchschnittlichen Intensitätsoszillationen der Reihe von Einzelbildern (201, 601) gebildet wird.

7. Verfahren gemäß einem der vorhergehenden Ansprüche 1 bis 5, **dadurch gekennzeichnet, dass** die Referenzfunktion (602) aus einem externen Signal gebildet wird, das den periodischen physiologischen Vorgang darstellt.

8. Verfahren gemäß Anspruch 6 oder 7, **dadurch gekennzeichnet, dass** das Ausgabebild aus der Reihe von Einzelbildern (201, 601) gebildet wird und dass jedes Einzelbild des Ausgabebildes durch Bilden der räumlichen Verteilung eines phasenverschobenen Korrelationsbildes gebildet wird, welches der Realteil des mit einem anderen Exponentialphasen-Koeffizienten multiplizierten Korrelationsbildes ist.

9. Verfahren gemäß Anspruch 8, **dadurch gekennzeichnet, dass** in dem Ausgabebild die räumliche Verteilung der Amplitude des Korrelationsbildes gezeigt wird.

10. Verfahren gemäß Anspruch 9, **dadurch gekennzeichnet, dass** die räumliche Verteilung der Amplitude im Ausgabebild mittels Pseudofarben kodiert ist, die die relative Phase des phasenverschobenen Korrelationsbildes darstellen.

11. Ein System zur Visualisierung von Pulsationswellen des Blutes in einem lebenden Körper, das Folgendes aufweist: mindestens eine Lichtquelle (102), die konfiguriert ist, um den lebenden Körper (101) zu beleuchten, eine Bilderfassungseinheit (106, 107) mit einem optischen Mittel (105) zum Sammeln des von dem lebenden Körper (101) reflektierten Lichts und zum Bilden eines fokussierten Bildes (201, 601) des lebenden Körpers (101) in der Bilderfassungseinheit (106, 107), eine Verarbeitungseinheit (109), eine Anzeigeeinheit (110) und eine Kommunikationsverbindung zwischen der Bilderfassungseinheit (106, 107), der Verarbeitungseinheit (109) und der Anzeigeeinheit (110), **dadurch gekennzeichnet, dass** die Verarbeitungseinheit (109) Mittel zum Empfangen der Reihe von Einzelbildern (201, 601) aufweist, die von der Bilderfassungseinheit (106, 107) erfasst wurden, und dass die Verarbeitungseinheit (109) Mittel zum Verarbeiten der Reihe von Einzelbildern (201, 601) gemäß einem der Ansprüche 1 bis 8 aufweist.

**12.** System gemäß Anspruch 11, **dadurch gekennzeichnet, dass** die Lichtquelle (102) eine Lichtquelle ist, die Licht im sichtbaren, im nahen infraroten oder infraroten Bereich des Spektrums erzeugt.

**13.** System gemäß Anspruch 11, **dadurch gekennzeichnet, dass** die Lichtquelle (102) eine Lichtquelle ist, die Licht in dem Pulsregime bei unterschiedlichen Wellenlängen synchron mit dem Framegrabber der Bilderfassungsvorrichtung (106, 107) erzeugt.

**Revendications**

**1.** Procédé de visualisation d'ondes de pulsations cardiovasculaires comprenant les étapes de procédé consistant à :

- éclairer avec une source de lumière (102) le corps vivant (101) avec une lumière pénétrant à travers la peau du corps (101) pour interagir par absorption et/ou par diffusion avec un système vasculaire du corps vivant (101),
- recueillir la lumière réfléchie par le corps vivant sous la forme d'une trame focalisée (201, 601) dans une unité de capture d'image (106, 107),
- capturer une série de trames (201, 601) par l'unité de capture d'image (106, 107) ;

**caractérisé en ce qu'**il comprend en outre les étapes consistant à :

- multiplier au moyen d'une unité de traitement (109) les trames (201, 601) de la série de trames par une fonction de référence (602) synchronisée avec un processus physiologique périodique du corps (101),
- former au moyen de l'unité de traitement (109) une image de corrélation en résumant des pixels respectifs multipliés par la fonction de référence (602) sur les trames (201, 601) de la série de trames,
- calculer au moyen de l'unité de traitement (109) une image de sortie représentant la dynamique des ondes de pulsations sanguines dans le corps vivant (101) à partir des images de corrélation en fonction de la phase du processus physiologique périodique du corps (101).

**2.** Procédé selon la revendication 1, **caractérisé en ce que** le processus physiologique périodique du corps (101) est les battements du coeur.

**3.** Procédé selon la revendication 1, **caractérisé en ce que** le processus physiologique périodique du corps (101) est la respiration.

**4.** Procédé selon l'une quelconque des revendications précédentes 1 à 3, **caractérisé en ce que** la lumière réfléchissante de la source lumineuse (102) est filtrée au moyen d'au moins deux filtres polarisants (103, 104), le premier filtre polarisant (103) étant positionné entre la source de lumière (102) et le corps vivant (101), et le second filtre polarisant (104) étant positionné entre le corps vivant éclairé (101) et l'unité de capture d'image (106, 107).

**5.** Procédé selon l'une quelconque des revendications précédentes 1 à 4, **caractérisé en ce que** les trames (201, 601) capturées par le dispositif de capture d'image (106, 107) ont une fréquence de trames au moins deux fois supérieure à la fréquence la plus élevée d'un processus physiologique quasi-périodique du corps vivant.

**6.** Procédé selon l'une quelconque des revendications précédentes 1 à 5, **caractérisé en ce que** la fonction de référence (602) est formée après l'analyse des oscillations d'intensité moyenne de la série de trames (201, 601).

**7.** Procédé selon l'une quelconque des revendications précédentes 1 à 5, **caractérisé en ce que** la fonction de référence (602) est formée à partir d'un signal externe représentant le processus physiologique périodique.

**8.** Procédé selon la revendication 6 ou la revendication 7, **caractérisé en ce que** l'image de sortie est formée à partir de séries de trames (201, 601) et **en ce que** chaque trame de l'image de sortie est formée en formant une distribution spatiale d'une image de corrélation déphasée qui est la partie réelle de l'image de corrélation multipliée par un coefficient de phase exponentielle différent.

**9.** Procédé selon la revendication 8, **caractérisé en ce que** dans l'image de sortie est représentée la distribution spatiale de l'amplitude de l'image de corrélation.

**10.** Procédé selon la revendication 9, **caractérisé en ce que** la distribution spatiale de l'amplitude dans l'image de sortie est codée au moyen de pseudo-couleurs représentant la phase relative de l'image de corrélation déphasée.

11. Système de visualisation d'ondes de pulsations sanguines dans un corps vivant comprenant au moins une source de lumière (102) configurée pour éclairer le corps vivant (101), une unité de capture d'image (106, 107) avec un moyen optique (105) pour recueillir la lumière réfléchie par le corps vivant (101) et former une image focalisée (201, 601) du corps vivant (101) dans l'unité de capture d'image (106, 107), une unité de traitement (109), une unité d'affichage (110), et une liaison de communication entre l'unité de capture d'image (106, 107), l'unité de traitement (109) et l'unité d'affichage (110), **caractérisé en ce que** l'unité de traitement (109) comprend un moyen pour recevoir la série de trames (201, 601) capturées par l'unité de capture d'image (106, 107) et **en ce que** l'unité de traitement (109) comprend un moyen pour traiter la série de trames (201, 601) selon l'une quelconque des revendications 1 à 8.

12. Système selon la revendication 11, **caractérisé en ce que** la source de lumière (102) est une source de lumière générant une lumière dans la région visible, proche infrarouge ou infrarouge du spectre.

13. Système selon la revendication 11, **caractérisé en ce que** la source de lumière (102) est une source de lumière générant une lumière dans le régime d'impulsions à différentes longueurs d'onde en synchronisme avec le moyen de capture de trames du dispositif de capture d'image (106, 107).

101

103 102 108 110

102

106 107

109

104

105

**Fig. 1**

201

202

**Fig. 2**

Fig. 3

319 — Time-series of images from 107

301 Selecting the reference area

302 Averaging pixels brightness in the reference area — $U_1(t)$

303 Applying FFT to the signal $U_1(t)$

304 Selecting cardiac frequencies

305 Applying inverse FFT — $R_C(t)$

306 Multiplying each frame by the reference $R_C(t)$

307 Summarizing the data in time for each pixel separately

308 Forming the image $Hc_0$ of the correlation amplitude

309 Forming phase shifted matrices $S_C^m$

310 Forming the output video for the cardiac cycle

311 Selecting respiratory frequencies

312 Applying inverse FFT — $R_B(t)$

313 Multiplying each frame by the reference $R_B(t)$

314 Summarizing the data in time for each pixel separately

315 Forming the image $Hb_0$ of the correlation amplitude

316 Forming phase shifted matrices $S_B^m$

317 Forming the output video for the respiratory cycle

318 — Time-series of images from 107

14

Fig. 4

Fig. 5

Fig. 6

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5747789 A
- WO 2007122375 A
- US 2008081968 A, S. Numada and T. Ozawa
- US 5983120 A, W. Groner and R. G. Nadeau
- WO 0215786 A, C. A. Cook, M. K. Emresoy, and H. Farid
- WO 0215788 A, E. P. Krotkov, Y. Wang, Z. Zhong, and R. M. Danen
- US 2005131284 A, A. Grinvald, D. Nelson, and I. Vanzetta

### Non-patent literature cited in the description

- **P. A. KYRIACOU.** Pulse oximetry in the oesophagus. *Physiol. Meas.,* 2006, vol. 27, R1-R35
- **J. ALLEN.** Photoplethysmography and its application in clinical physiological measurement. *Physiol. Meas.,* 2007, vol. 28, R1-R40
- **K. HUMPHREYS ; T. WARD ; C. MARKHAM.** Non-contact simultaneous dual wavelength photoplethysmography: a further step toward noncontact pulse oximetry. *Rev. Sci. Instrum.,* 2007, vol. 78, 044304-1, 044304-6
- **F. P. WIERINGA ; F. MASTIK ; A. F. W. VAN DER STEEN.** Contactless multiple wavelength photoplethysmographic imaging: a first step toward "SpO2 camera" technology. *Ann. Biomed. Eng.,* 2005, vol. 33, 1034-1041
- **W. CUI ; L. E. OSTRANDER ; B. Y. LEE.** In vivo reflectance of blood and tissue as a function of light wavelength. *IEEE Trans. Biomed. Eng.,* 1990, vol. 37, 632-639